# EUROPEAN PATENT APPLICATION

(11) **EP 4 129 415 A1**
(43) Date of publication of application: **08.02.2023**
(21) Application number: 22188264.0
(22) Date of filing: 02.08.2022
(51) Int. Cl.: A61Q 17/04, A61K 8/92, A61K 8/9717, A61K 8/9789

(54) **SUN PROTECTION COMPOSITION**

(30) Priority: 03.08.2021 GB 202111197
(71) Applicant: Lawrence, Clare, Chippenham Wiltshire SN14 7BH (GB)
(72) Inventor: Lawrence, Clare, Chippenham Wiltshire SN14 7BH (GB)
(74) Representative: Barker Brettell LLP

(57) **Abstract**

A sun protection composition comprising: sesame seed oil; rhatany root extract; and seaweed extract, wherein the composition is free of zinc oxide.

## Description

### Field of the invention

The present invention relates generally to sun protection compositions which do not comprise zinc oxide.

### Background of the invention

Aside from their beautiful aesthetics, coral reefs are integral to the survival of many species of marine life and precise functioning of the ocean's ecosystem. Studies have shown a drastic decline in many forms of coral over the last decade and, because of climate change and certain destructive human practices, this decline is unfortunately set to continue. The extinction of coral reefs would be particularly damaging for those countries and people who rely on them for their livelihood e.g. through tourism and the fishing industry.

There are several causes of coral destruction ranging from careless tourist behaviour to changes in water temperature due to global warming. Another cause is the chemicals found in sunscreen products.

There are broadly two types of sunscreen products available for use - chemical sunscreens and physical sunscreens. Chemical sunscreens (also known as organic sunscreens) use filtering agents such as avobenzone, octinoxate and oxybenzone to absorb the sun's UV rays. Physical sunscreens (also known as inorganic sunscreens) use mineral-based filtering agents such as zinc oxide and titanium oxide to deflect or block the sun's UV rays.

It is generally known that the ingredients found in chemical sunscreen products can cause serious damage to coral reefs and marine life, having been shown to have direct links to coral bleaching. There has also been controversy surrounding the use of nanoparticle-based mineral ingredients such as nano-zinc oxide which are also damaging to coral reefs.

Aside from the detrimental effects on coral, the ingredients used in chemical sunscreens can be irritating to human skin and cause allergic reactions. There are also safety concerns surrounding the use of nanoparticle ingredients in physical sunscreens which may be absorbed by your skin and enter the bloodstream.

Therefore, non-nanoparticle-based physical sunscreens, particularly those containing zinc oxide, are widely used and encouraged, being FDA-approved and branded as human and coral reef safe.

However, it is now known that zinc oxide and other mineral ingredients, whether they be nano-sized or not, contribute to coral reef damage. Therefore, there is an urgent need for sun protection compositions that are coral reef safe as well as being safe and protective for humans.

### Summary of the invention

Accordingly, in a first aspect of the invention, there is provided a sun protection composition comprising:
(i) sesame seed oil;
(ii) rhatany root extract; and
(iii) seaweed extract,
wherein the composition is free of zinc oxide.

In another aspect of the invention, there is provided a method for protecting the skin and/or hair from UV radiation comprising applying a sun protection composition to the skin and/or hair, wherein the composition comprises:
(i) sesame seed oil;
(ii) rhatany root extract; and
(iii) seaweed extract,
wherein the composition is free of zinc oxide.

In another aspect of the invention, there is provided a use of a sun protection composition to protect the skin and/or hair from UV radiation, wherein the composition comprises:
(i) sesame seed oil;
(ii) rhatany root extract; and
(iii) seaweed extract,
wherein the composition is free of zinc oxide.

The present invention is characterised by a specific combination of ingredients which together provide protection against UV radiation without the need for zinc oxide. Advantageously, in the absence of zinc oxide, the problems associated with this mineral agent are overcome, leading to sunscreen products which are less or not harmful to coral reefs. The ingredients used (and not used) additionally lead to products which are well tolerated on the skin and may reduce or prevent the allergic or irritating effects often associated with conventional sunscreen products.

### Detailed description of the invention

The invention makes use of sesame seed oil. The sesame seed oil may be *Sesamum indicum* seed oil, i.e. oil obtained from obtained from the seeds of *Sesamum indicum* (CAS number 8008-74-0).

The sesame seed oil may be present in an amount of from about 0.1% to 10% by weight relative to the total weight of the composition, for example from about 1% to about 9% by weight of the composition, or from about 3% to about 8% by weight of the composition. The sesame seed oil may be present in an amount of about 1% by weight relative to the total weight of the composition, for example in an amount of at least about 2%, at least about 2.5%, at least about 3%, at least about 4%, at least about 5%, at least about 6%, at least about 7%, at least about 7.5%, at least about 8%, at least about 9% or at least about 10% by weight of the composition. In one embodiment, the sesame seed oil, e.g. *Sesamum indicum* seed oil, is present at a level of about 7% to 8% by weight relative to the total weight of the composition.

The invention makes use of rhatany root extract. The rhatany root extract may be *Krameria triandra* root extract (CAS number 84775-95-1). The rhatany root extract may be present in an amount of from about 0.1% to 10% by weight relative to the total weight of the composition, for example from about 0.5% to about 7.5% by weight of the composition, or from about 1% to about 5% by weight of the composition, or from about 1% to about 3% by weight of the composition, or from about 0.5% to about 2.5% by weight of the composition. The rhatany root extract may be present in an amount of at least about 0.1%, at least about 0.5%, at least about 0.75%, at least about 1%, at least about 1.5%, at least about 2%, or at least about 2.5% by weight relative to the total weight of the composition. In one embodiment, the rhatany root extract e.g. *Krameria triandra* root extract, is present at a level of about 2% to 3% by weight relative to the total weight of the composition.

The sesame seed oil and rhatany root extract may be present in the composition in a ratio of about 3: about 1, respectively.

The invention makes use of a seaweed extract. The extract may be derived from a seaweed of *the genus Porphyra.* For example, the seaweed extract may be *Porphyra akasakae, Porphyra angusta, Porphyra argentinensis, Porphyra atropurpurea, Porphyra augustinae, Porphyra autumnalis, Porphyra bangiaeformis, Porphyra bulbopes, Porphyra capensis Porphyra carnea, Porphyra ceylanica, Porphyra chauhanii, Porphyra corallicola, Porphyra cordata, Porphyra cucullata, Porphyra delicatula, Porphyra dentimarginata, Porphyra dioica, Porphyra drewiae, Porphyra fujianensis, Porphyra grateloupicola, Porphyra grayana, Porphyra guangdongensis, Porphyra haitanensis, Porphyra hospitans, Porphyra inaequicrassa, Porphyra indica, Porphyra ionae, Porphyra irregularis, Porphyra kanyakumariensis, Porphyra laciniata, Porphyra ledermannii, Porphyra linearis, Porphyra lucasii, Porphyra maculosa, Porphyra malvanensis, Porphyra marcosii, Porphyra marginata, Porphyra martensiana, Porphyra microphylla, Porphyra minima, Porphyra minor, Porphyra monosporangia, Porphyra mumfordii, Porphyra njordii, Porphyra nobilis, Porphyra ochotensis, Porphyra okamurae, Porphyra okhaensis, Porphyra oligospermatangia, Porphyra plocamiestris, Porphyra pujalsiae, Porphyra pulchra, Porphyra punctata, Porphyra purpurea, Porphyra qingdaoensis, Porphyra ramosissima, Porphyra reniformis, Porphyra rizzinii, Porphyra roseana, Porphyra schistothallus, Porphyra segregata, Porphyra tenuis, Porphyra tenuissima, Porphyra tristanensis, Porphyra umbilicalis, Porphyra umbilicata, Porphyra violacea, Porphyra vulgaris, Porphyra woolhouseae, Porphyra yamadae,* or any combination thereof. In one embodiment, the seaweed extract is a *Porphyra umbilicalis* extract.

The seaweed extract may be present in an amount of from about 0.1% to 10% by weight relative to the total weight of the composition, for example from about 0.5% to about 7.5% by weight of the composition, or from about 0.5% to about 3% by weight of the composition, or from about 0.75% to about 2.5% by weight of the composition. The seaweed extract may be present in an amount of at least about 0.1%, at least about 0.5%, at least about 0.75%, at least about 1%, at least about 1.25%, or at least about 1.5% by weight relative to the total weight of the composition. In one embodiment, the seaweed extract, e.g. *Porphyra umbilicalis* extract, is present at a level of about 2% to 3% by weight relative to the total weight of the composition.

The composition may further comprise a rosemary extract. The extract may be a *Rosmarinus officinalis* extract (also known as a *Salvia rosmarinus* extract). The extract may be a *Rosmarinus officinalis* leaf extract.

The rosemary extract may be present in an amount of from about 0.01% to 10% by weight relative to the total weight of the composition, for example from about 0.01% to about 1% by weight of the composition, or from about 0.05% to about 0.75% by weight of the composition, or from about 0.1% to about 0.6% by weight of the composition, or from about 0.2% to about 0.5% by weight of the composition, or from about 0.25% to about 0.4% by weight of the composition. In one embodiment, the rosemary extract, e.g. *Rosmarinus officinalis* leaf extract, is present at a level of about 0.3% to 0.4% by weight relative to the total weight of the composition.

The sun protection composition of the invention may further comprise olive oil. The olive oil may be *Olea europaea* fruit oil.

The olive oil may be present in an amount of from about 40% to about 90% by weight relative to the total weight of the composition, for example from about 50% to about 85% by weight of the composition, for example from about 60% to about 80% by weight of the composition, from about 62% to about 76% by weight of the composition, from about 65% to about 75% by weight of the composition, from about 67% to about 74% by weight of the composition or from about 69% to about 73% by weight of the composition. The olive oil may be present in an amount from at least about 10% by weight of the composition, at least about 20%, at least about 25%, at least about 30%, at least about 33%, at least about 40%, at least about 45%, at least about 50%, at least about 55%, at least about 60%, at least about 65%, at least about 70% by weight of the composition.

The sun protection composition of the invention may further comprise honeysuckle flower extract. The honeysuckle flower extract may be derived from a honeysuckle flower of the genus *Lonicera,* for example *Lonicera japonica* and/or *Lonicera caprifolium.* When present, the honeysuckle flower extract may be present in an amount of from about 0.01% to 10% by weight relative to the total weight of the composition, for example from about 0.1% to about 5% by weight of the composition, or from about 0.5% to about 1% by weight of the composition.

The sun protection composition of the invention is free of zinc oxide. In other words, the composition does not comprise or it excludes zinc oxide. The term "zinc oxide" is intended to cover any form of zinc oxide, e.g. zinc oxide, nanoparticle zinc oxide, surface-treated zinc oxide, zinc oxide in a powder form, zinc oxide in a dispersion, zinc oxide in a slurry form, zinc oxide in a paste form.

In one embodiment, the sun protection composition of the invention may also be free of other mineral-based UV protective agents (in addition to zinc oxide). In other words, the composition may not comprise or it excludes one or more further, or any, mineral-based sunscreen actives/inorganic sunscreen agents. In one embodiment, the composition is free of all mineral-based UV protective agents. The term "mineral-based UV protective agents" includes all well-known mineral sunscreen actives found in conventional physical/inorganic sunscreens. For example zinc oxide, nanoparticle zinc oxide, titanium dioxide, nanoparticle titanium dioxide, iron oxide, talcs and/or boron nitride. In one embodiment, the sun protection composition does not comprise any form of zinc oxide and any form of titanium dioxide.

In one embodiment, the sun protection composition is also free of chemical-based UV protective agents. In other words, in some embodiments the sun protection composition does not comprise one or more, or any, chemical-based sunscreen actives/organic sunscreen agents. In one embodiment, the composition is free of all chemical-based UV protective agents. The term "chemical-based UV protective agents" includes all well-known chemical sunscreen actives found in conventional chemical/organic sunscreens. For example p-aminobenzoic acids, esters and derivatives thereof (e.g. 2-ethylhexyl p-dimethyl-aminobenzoate), methoxycinnamate esters (e.g., octinoxate, 2-ethylhexyl p-methoxycinnamate, 2-ethoxyethyl p-methoxycinnamate or α,β-di-(p-methoxycinnamoyl)-α'-(2ethylhexanoyl)-glycerin), benzophenones (e.g. avobenzone oxybenzone), dibenzoylmethanes (e.g. 4-(tert-butyl)-4'-methoxydibenzoylmethane), 2-phenylbenzimidazole-5 sulfonic acid and salts thereof, alkyl-β,β-diphenylacrylates (e.g. alkyl α-cyano-β,β-diphenylacrylates such as octocrylene) triazines (such as 2,4,6-trianilino-(p-carbo-2-ethyl-hexyl-1-oxy)-1,3,5 triazine), and/or camphor derivatives (such as methylbenzylidene camphor).

In one embodiment, the sun protection composition of the invention provides protection against at least about 10% of UVA and/or UVB rays, or at least about 15%, or at least about 20%, or at least about 25%, or at least about 30%, or at least about 35%, or at least about 40%, or at least about 50%, or at least about 60%, or at least about 70%, or at least about 75%, or at least about 80%, or at least about 90%, or at least about 95% of UVA and/or UVB rays.

In some embodiments, the sun protection composition further comprises a cosmetically acceptable carrier. The carrier may be water-based, oil-based, or emulsion-based.

In embodiments where the carrier is emulsion-based, the composition may be in the form of a water-in-oil, an oil-in-water, a water-in-oil-in-water or a oil-in-water-in-oil emulsion.

In embodiments where the sun protection composition is in the form of a water-in-oil emulsion, water may be present at a level of about 0.1% to about 20% by weight of the composition, about 0.5% to about 10% by weight of the composition, or about 1% to about 5% by weight of the composition. In one embodiment where the cosmetic composition is in the form of a water-in-oil emulsion, water is present at a level of about 1.5% to about 2.5% by weight of the composition.

The sun protection composition of the invention may be provided in any form suitable for topical application to the skin and/or hair. The cosmetic composition of the invention may be delivered and/or applied to the skin and/or hair via any of the conventional formulations known to those skilled in the art. Typical formulation types of the present invention are liquids, creams, lotions, milks, mousses, gels, sprays, serum, foams, aerosols, and ointments. In one embodiment, the sun protection composition of the invention is formulated as a sunscreen product (such as a sunscreen lotion, a sunscreen spray), a cosmetic product (such as a moisturiser, a face cream, an eye-cream) and/or a hair-care product (such as a shampoo, a conditioner).

The sun protection composition of the invention will generally further comprise other ingredients or excipients which will be well known to those skilled in the art.

The sun protection composition of the invention may further comprise one or more humectants, including but not limited to betaine, glycerin, propylene glycol, butylene glycol, hexylene glycol, dipropylene glycol, polyethylene glycol, sorbitol, sodium hyaluronate, urea, xylitol, lactate, fructose, glucose, mannose, xylose, honey, pyrrolidone, and carboxylic acid and salts thereof. When present, the one or more humectants may be present in the cosmetic composition in an amount of about 0.01% to about 20% by weight of the composition, about 0.1% to about 10%, or about 0.5% to about 7% by weight of the composition.

The sun protection composition of the invention may further comprise one or more emollients, including but not limited to Polyglyceryl-2 diisostearate, Sucrose tetrastearate, triacetatecetyl esters, cera alba, PPG-15 stearyl ether, ethylhexyl stearate, cetyl dimethicone, octyldodecanol, PPG-20 methyl glucose ether, isopropyl myristate, isopropyl paltimate, isopropyl laurate, isodecyl laurate, isodecyl neopentanoate, isohexadecane, pentaerythrityl tetraisostearate, caprylic/capric triglyceride, canola oil, sunflower oil *(Helianthus annus),* olive oil *(Olea europea),* cottonseed oil *(Gossypium herbaceum),* jojoba oil *(Simmondsia chinensis),* shea butter *(Butyrospermum parkii),* cocoa butter *(Theobroma cacao),* cupuacu butter *(Theobroma grandiflorum),* avocado oil *(Persea gratissima),* liquid paraffin, dimethicone, phenyl trimethicone, cyclopentasiloxane, dimethiconol and petrolatum. When present, the one or more emollients may be present in the cosmetic composition in an amount of about 0.01% to about 20% by weight of the composition, about 0.1% to about 10%, or about 0.5% to about 7% by weight of the composition.

The sun protection composition may further comprise one or more emulsifiers, including but not limited to steareth-2, steareth-21, steareth-10, ceteareth-5, ceteareth-20, cetearyl glucoside, oleth-10, glyceryl stearate, polyglycerol-3 oleate, polyglyceryl-3 methylglucose distearate, sodium stearate, PEG-60, PEG-12 oleate, PEG-2 stearate, PEG-12 stearate, PEG-20 stearate, PEG-100 stearate, polysorbate-60, cetyl alcohol, cetearyl alcohol, potassium cetyl phosphate, cetearyl olivate, sorbitan olivate, PEG-80 sorbitan, sorbitan oleate, sorbitan stearate, and/or sorbitan palmitate. In one embodiment, the sun protection composition of the invention does not comprise sulphates as emulsifiers. In embodiments where one or more emulsifiers are present in the cosmetic composition, the one or more emulsifiers may be present in an amount of about 0.01% to about 5% or about 0.01% to about 2% by weight of the composition. In one embodiment, the cosmetic composition of the invention does not contain emulsifiers.

The sun protection composition of the invention may further comprise one or more surfactants, including but not limited to, anionic surfactants (e.g. sodium lauryl sulphate, sodium laureth sulphate, ammonium laureth sulphate, disodium laureth sulfosuccinate and sodium C12-15 pareth-12 carboxylate), amphoteric/zwitterionic surfactants (e.g. cocamidopropyl betaine, sodium cocoamphoacetate and cocamidopropyl hydroxysultaine), non-ionic surfactants (e.g. cocamide DEA, cocamide MEA, decyl glucoside, lauryl glucoside), and cationic surfactants (e.g. cetrimonium chloride, behentrimonium chloride and benzalkonium chloride). In embodiments where one or more surfactants are present in the sun protection composition, the one or more surfactants may be present in an amount of from about 0.01% to about 10% by weight of the composition, e.g. from about 0.25% to about 7.5% by weight of the composition, or about 0.5% to about 6% by weight of the composition. In one embodiment where one or more surfactants are present in the sun protection composition, the one or more surfactants are present in an amount of from about 0.5% to about 5% by weight of the composition.

The sun protection composition of the invention may further comprise one or more preservatives, including but not limited to, ascorbyl palmitate, 2-bromo-2nitropropane-1,3-diol (bronopol, commercially available under the trade name Myacide RTM), benzyl alcohol, benzoic acid, sodium benzoate, diazolidinyl urea, imidazolidinyl urea, methyl paraben, phenoxyethanol, ethyl paraben, propyl paraben, sodium methyl paraben, sodium dehydroacetate, dehydroacetic acid, polyhexamethylenebiguanide hydrochloride, isothiazolone, chlorhexidine digluconate, chlorphensin and/or sodium propyl paraben. In embodiments where one or more preservatives are present in the sun protection composition, the one or more preservatives may be present in an amount of about 0.001% to about 10% by weight of the composition, about 0.01% to about 8% by weight of the composition, or about 0.1% to about 5% by weight of the composition. In one embodiment, the preservatives are sodium benzoate and/or potassium sorbate. In one embodiment where one or more preservatives are present in the sun protection composition, the one or more preservatives are present in an amount of about 0.05% to about 8% by weight of the composition.

The sun protection composition of the invention may further comprise one or more chelating agents or sequestering agents, including but not limited to, ethylenediamine tetraacetic acid (EDTA) and salts thereof (e.g. dipotassium EDTA, disodium EDTA or tetrasodium EDTA), sodium phytate, trisodium ethylene diamine disuccinate, and/or tetrasodium glutamate diacetate. In embodiments where one or more chelating agents are present in the sun protection composition, the one or more chelating agents may be present in an amount of about 0.001% to about 10% by weight of the composition, about 0.01% to about 8% by weight of the composition, or about 0.1% to about 5% by weight of the composition. In one embodiment where one or more chelating agents are present in the sun protection composition, the one or more chelating agents are present in an amount of about 0.05% to about 8% by weight of the composition.

The sun protection composition of the invention may further comprise one or more vitamins. For example, the cosmetic composition may further comprise vitamin B, vitamin B1 to vitamin B12, vitamin C, vitamin D, vitamin E, vitamin K, vitamin H, derivatives thereof, provitamins thereof (e.g. provitamin B5 (panthenol)), or combinations thereof. In embodiments where one or more vitamins are present in the sun protection composition, the one or more vitamins may be present in an amount of about 0.0001% to about 50% by weight of the composition, about 0.001% to about 10% by weight of the composition, about 0.01% to about 8% by weight of the composition, or about 0.1% to about 5% by weight of the composition. In one embodiment where one or more vitamins are present in the sun protection composition, the one or more vitamins are present in an amount of about 0.1% to about 5% by weight of the composition.

The sun protection composition of the invention may further comprise one or more pH adjusting agents, including but not limited to lactic acid, potassium hydroxide, sodium hydroxide, aminomethyl propanol, citric acid, sodium citrate, and/or triethanolamine. The sun protection composition of the invention may have a pH from about 3 to about 10, e.g. from about 4 to about 8, or from about 5 to about 7. In embodiments where one or more pH adjusting agents are present in the sun protection composition, the one or more pH adjusting agents may be present in an amount of from about 0.001 to about 10% by weight of the composition, preferably about 0.001 to 0.5% by weight of the composition.

The sun protection composition of the invention may further comprise one or more thickeners or gelling agents. In one embodiment, the thickener is selected from the group consisting of Cocamide MEA, Glyceryl Laurate, Glyceryl Oleate, Laureth-3, Laureth-4, PEG-7 Glyceryl Cocoate, PEG-18 Glyceryl Oleate, PEG-18 Glyceryl Cocoate, PEG-120 Methyl Glucose Dioleate, PEG-120 Methyl Glucose Trioleate, PEG-150 Distearate, PEG-200 Hydrogenated Glyceryl Palmate, Acrylates Copolymer, Acrylates/C10-30 Alkyl Acrylate Crosspolymer, Acrylates Crosspolymer-4, Carbomer, Hydroxyethyl Cellulose, Magnesium Aluminium Silicate, Xanthan Gum, or combinations thereof. In one embodiment, the thickener is selected from the group consisting of Cocamide MEA, Glyceryl Laurate, Glyceryl Oleate, Laureth-3, Laureth-4, PEG-7 Glyceryl Cocoate, PEG-18 Glyceryl Oleate, PEG-18 Glyceryl Cocoate, PEG-120 Methyl Glucose Dioleate, PEG-120 Methyl Glucose Trioleate, PEG-150 Distearate, PEG-200 Hydrogenated Glyceryl Palmate, or combinations thereof. In embodiments where one or more thickeners/gelling agents are present in the sun protection composition, the one or more thickeners/gelling agents may be present in an amount of about 0.01 to about 10% by weight of the composition.

The sun protection compositions of the invention may further comprise one or more perfumes and/or colourings.

In the present application, the term "about" may encompass ±10%, such as ±5%, e.g. ±2% or ±1%.

The skilled person will understand that optional features of one embodiment or aspect of the invention may be applicable, where appropriate, to other embodiments or aspects of the invention.

### Other embodiments

In one embodiment, there is provided a sun protection composition comprising:
(i) sesame seed oil;
(ii) rhatany root extract;
(iii) seaweed extract; and
(iv) olive oil,
wherein the composition is free of zinc oxide.

In one embodiment, there is provided a sun protection composition comprising:
(i) sesame seed oil;
(ii) rhatany root extract;
(iii) seaweed extract;
(iv) olive oil; and
(v) rosemary leaf extract,
wherein the composition is free of zinc oxide.

In one embodiment, there is provided a sun protection composition comprising:
(i) 1-10% (w/w) sesame seed oil;
(ii) 1-5% (w/w) rhatany root extract;
(iii) 1-5% (w/w) seaweed extract;
(iv) 50-80% (w/w) olive oil; and
(v) 0.1%-1% (w/w) rosemary leaf extract,
wherein the composition is free of zinc oxide.

In one embodiment, there is provided a sun protection composition comprising:
(i) 1-10% (w/w) sesame seed oil;
(ii) 1-5% (w/w) rhatany root extract;
(iii) 1-5% (w/w) seaweed extract;
(iv) 50-80% (w/w) olive oil; and
(v) 0.1%-1% (w/w) rosemary leaf extract,
wherein the composition is free of zinc oxide and titanium dioxide.

In one embodiment, there is provided a sun protection composition comprising:
(i) sesame seed oil;
(ii) rhatany root extract;
(iii) seaweed extract;
(iv) olive oil;
(v) rosemary leaf extract; and
(vi) honeysuckle flower extract
wherein the composition is free of zinc oxide.

In one embodiment, there is provided a consumer product comprising a sun protection composition of the invention, wherein the consumer product is selected from the group consisting of a sunscreen product (e.g. a sunscreen lotion, a sunscreen spray), a cosmetic product (e.g. a moisturiser, a face cream, an eye-cream), a make-up product (e.g. a foundation), and a hair-care product (e.g. a shampoo, a conditioner).

In one embodiment, there is provided a composition comprising:
(i) sesame seed oil (e.g. 1-10% (w/w));
(ii) rhatany root extract (e.g. 1-5% (w/w));
(iii) seaweed extract (e.g. 1-5% (w/w)); and
(iv) olive oil (e.g. 50-80% (w/w)); and optionally
(v) rosemary leaf extract (e.g. 0.1-1% (w/w)) and/or honeysuckle flower extract (e.g. 0.1-1% (w/w)),
wherein the composition is free of zinc oxide.

### Example formulation

| | |
|---|---|
| *Olea europaea* (Olive) fruit oil | 70-75% (w/w) |
| Sucrose tetrastearate triacetate | 5-10% (w/w) |
| *Sesamum indicum* (Sesame) seed oil | 5-10% (w/w) |
| Polyglyceryl-2 diisostearate | 1-5% (w/w) |
| *Krameria triandra* root extract | 1-5% (w/w) |
| Aqua [Water] | 1-5% (w/w) |
| *Porphyra umbilicalis* extract | 1-5% (w/w) |
| *Lonicera japonica* (Honeysuckle) flower extract | 0.5-2% (w/w) |
| Lactic acid | 0.1-1% (w/w) |
| *Rosmarinus officinalis* (Rosemary) leaf extract | 0.1-1% (w/w) |
| *Lonicera caprifolium* (Honeysuckle) flower extract | 0.1-1% (w/w) |
| Betaine | 0.1-1% (w/w) |
| Sodium benzoate | 0.01-0.5% (w/w) |
| Potassium sorbate | 0.001-0.05% (w/w) |
| Ascorbyl palmitate | 0.001-0.05% (w/w) |
| Citric acid | 0.001-0.05% (w/w) |
| Glyceryl oleate | 0.001-0.05% (w/w) |
| Glyceryl stearate | 0.001-0.05% (w/w) |
| Lecithin | 0.001-0.05% (w/w) |
| Tocopherol | 0.001-0.05% (w/w) |

## Claims

1. A sun protection composition comprising:
(i) sesame seed oil;
(ii) rhatany root extract; and
(iii) seaweed extract;
wherein the composition is free of zinc oxide.

2. The sun protection composition of claim 1, further comprising olive oil and/or rosemary leaf extract.

3. The sun protection composition of any one of the preceding claims, wherein the sesame seed oil is *Sesamum indicum* seed oil, the rhatany root extract is *Krameria triandra* root extract, the seaweed extract is *Porphyra umbilicalis* extract, the olive oil is *Olea europaea* fruit oil and the rosemary leaf extract is *Rosamarinus officinalis* leaf extract.

4. The sun protection composition of any one of the preceding claims, wherein the sesame seed oil is present in an amount of about 1% to about 10% by weight of the total weight of the composition, or about 5% to about 8.5% by weight of the total weight of the composition.

5. The sun protection composition of any one of the preceding claims, wherein the rhatany root extract is present in an amount of about 0.5% to about 10% by weight of the total weight of the composition, or about 1% to about 4.5% by weight of the total weight of the composition.

6. The sun protection composition of any one of the preceding claims, wherein the seaweed extract is present in an amount of about 0.1% to about 10% by weight of the total weight of the composition, or about 0.25% to about 4.5% by weight of the total weight of the composition.

7. The sun protection composition of any one of claims 2 to 6, wherein the olive oil is present in an amount of about 40% to about 85% by weight of the total weight of the composition, or about 60% to about 80% by weight of the total weight of the composition; and/or wherein the rosemary leaf extract is present in an amount of about 0.01% to about 3% by weight of the total weight of the composition, or about 0.05% to about 0.9% by weight of the total weight of the composition.

8. The sun protection composition of any one of the preceding claims, wherein the composition is also free of titanium dioxide, and/or wherein the composition is free of any mineral-based UV protective agents and/or wherein the composition is free of any chemical-based UV protective agents.

9. The sun protection composition of claim 9, wherein the composition the mineral-based UV protective agent is selected from the group consisting of nanoparticle zinc oxide, titanium dioxide, nanoparticle titanium dioxide, iron oxide, talcs and/or boron nitride; and/or
wherein the chemical-based UV protective agent is selected from the group consisting of p-aminobenzoic acids, esters and derivatives thereof (e.g. 2-ethylhexyl p-dimethyl-aminobenzoate), methoxycinnamate esters (e.g., octinoxate, 2-ethylhexyl p-methoxycinnamate, 2-ethoxyethyl p-methoxycinnamate or α,β-di-(p-methoxycinnamoyl)-α'-(2ethylhexanoyl)-glycerin), benzophenones (e.g. avobenzone oxybenzone), dibenzoylmethanes (e.g. 4-(tert-butyl)-4'-methoxydibenzoylmethane), 2-phenylbenzimidazole-5 sulfonic acid and salts thereof, alkyl-β,β-diphenylacrylates (e.g. alkyl α-cyano-β,β-diphenylacrylates such as octocrylene) triazines (such as 2,4,6-trianilino-(p-carbo-2-ethyl-hexyl-1-oxy)-1,3,5 triazine), and/or camphor derivatives (such as methylbenzylidene camphor)

10. The sun protection composition of any one of the preceding claims, further comprising honeysuckle flower extract, sucrose tetrastearate triacetate and/or polyglyceryl-2-diisostearate.

11. A method for protecting the skin and/or hair from UV radiation comprising applying a sun protection composition as defined in any one of the preceding claims to the skin and/or hair.

12. Use of a sun protection composition as defined in any one of claims 1 to 11, to protect the skin and/or hair from UV radiation.

13. A consumer product comprising a sun protection composition as defined in any one of claims 1 to 11, wherein the consumer product is selected from the group consisting of a sunscreen product, a cosmetic product, a make-up product, a haircare product, and combinations thereof.

14. The consumer product of claim 14, wherein the sun protection composition is present in an amount of about 1% to about 10% by weight of the total weight of the consumer product.

15. A composition comprising:
(i) 1-12% (w/w) sesame seed oil;
(ii) 1-5% (w/w) rhatany root extract;
(iii) 1-5% (w/w) seaweed extract; and
(iv) 50-80% (w/w) olive oil,
wherein the composition is free of zinc oxide.
